(19) Europäisches Patentamt
European Patent Office
Office européen des brevets

(11) Publication number: **0 199 524 B1**

(12) **EUROPEAN PATENT SPECIFICATION**

(45) Date of publication of patent specification: **05.02.92** (51) Int. Cl.[5]: **C07F 15/00**, A61K 31/28

(21) Application number: **86302787.6**

(22) Date of filing: **15.04.86**

# (54) Platinum tramine antitumor agents.

(30) Priority: **16.04.85 US 723783**

(43) Date of publication of application:
**29.10.86 Bulletin 86/44**

(45) Publication of the grant of the patent:
**05.02.92 Bulletin 92/06**

(84) Designated Contracting States:
**AT BE CH DE FR GB IT LI LU NL SE**

(56) References cited:
**EP-A- 0 115 929**

**CHEMICAL ABSTRACTS, vol. 80, no. 10, 11th March 1974, page 540, abstract no. 55434b, Columbus, Ohio, US; Yu.N. KUKUSHKIN et al.: "Thermal reaction of pyridine- and beta-picolineethylenediaminetetramines of platinum (II)", & ZH. OBSHCH. KHIM. 1973, 43(11), 2352-3**

**CHEMICAL ABSTRACTS, vol. 82, no. 4, 27th January 1975, page 501, abstract no. 24033g, Columbus, Ohio, US; S.T. CHOW et al.: "Carbon-13-shieldings and couplings with platinum-195 in pyridine and bipyridyl complexes", & INORG. NUCL. CHEM. LETT. 1974, 10(12), 1131-5**

**CHEMICAL ABSTRACTS, vol. 96, no. 21, 24th May 1982, page 700, abstract no. 181154d, Columbus, Ohio, US; & JP-A-81 158 763 (SANKYO CO. LTD) 07-12-1981**

(73) Proprietor: **ENGELHARD CORPORATION**
**70 Wood Avenue South CN 770**
**Iselin New Jersey 08830(US)**

(72) Inventor: **Hollis, Leslie S.**
**33 Guilford Lane**
**Mercerville New Jersey 08619(US)**
Inventor: **Amundsen, Alan R.**
**12 Meadowbrook Drive**
**Somervile New Jersey 08876(US)**
Inventor: **Stern, Eric W.**
**234 Oak Tree Road**
**Mountainside New Jersey 07092(US)**

(74) Representative: **Fisher, Adrian John et al**
**CARPMAELS & RANSFORD 43 Bloomsbury Square**
**London WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 199 524 B1

## Description

### Field of the Invention

This invention relates to platinum(II) triamine complexes which have high aqueous solubility and aqueous stability and display antitumor activity.

### Brief Description of the Drawings

Figure 1 is a reproduction of the infrared spectrum of cis-chlorodiamminepyridineplatinum(II) chloride, cis-[Pt($NH_3$)$_2$(py)Cl]Cl, Example 1.

Figure 2 is a reproduction of the infrared spectrum of cis-chlorodiammine-2-hydroxypyridineplatinum(II) chloride, cis-[Pt($NH_3$)$_2$(2-pyOH)Cl]Cl, Example 2.

Figure 3 is a reproduction of the infrared spectrum of cis-chlorodiammine-6-methyl-2-hydroxypyridine-platinum(II) chloride, cis-[Pt($NH_3$)$_2$(6-Me-2-pyOH)Cl]Cl, Example 3.

Figure 4 is a reproduction of the infrared spectrum of cis-chlorodiammine-3-hydroxypyridineplatinum(II) chloride, cis-[Pt($NH_3$)$_2$(3-pyOH)Cl]Cl, Example 4.

Figure 5 is a reproduction of the infrared spectrum of cis-chlorodiammine-4-methylpyridineplatinum(II) chloride, cis-[Pt($NH_3$)$_2$(4-$CH_3$-Py)Cl]Cl, Example 5.

Figure 6 is a reproduction of the infrared spectrum of cis-chlorodiamminecytosineplatinum(II) chloride, cis-[Pt($NH_3$)$_2$(cytosine)Cl]Cl, Example 6.

Figure 7 is a reproduction of the infrared spectrum of cis-chlorodiammine-6-methyl-3-hydroxypyridine-platinum(II) chloride, cis-[Pt($NH_3$)$_2$(6-Me-3-pyOH)Cl]Cl, Example 7.

Figure 8 is a reproduction of the infrared spectrum of cis-chlorodiammine-4-chloropyridine-platinum(II) chloride, cis-[Pt($NH_3$)$_2$(4-Cl-py)Cl]Cl, Example 8.

Figure 9 is a reproduction of the infrared spectrum of trans-chlorodiamminepyridineplatinum(II) chloride, trans-[Pt($NH_3$)$_2$(py)Cl]Cl, Example 18.

### Background

The discovery, development, and subsequent commercialization of the antitumor agent cisplatin (cis-diamminedichloroplatinum(II)) has resulted in expanded interest in metal coordination compounds as medicinal agents over the past decade and one half. Platinum compounds, in particular, have received considerable scrutiny as antineoplastics as part of an ongoing search for second and third generation anologs of cisplatin. Among the goals of this research has been platinum compounds showing greater or different activity or potency than cisplatin and platinum compounds having superior physical and chemical properties than cisplatin. Of particular interest in the latter respect are aqueous solubility and stability. We describe here a series of platinum compounds, in particular, platinum(II) triamine complexes, some novel, which show antitumor activity and both high water solubility and excellent solution stability.

Some platinum triamine compounds have been reported in the literature. These include cis-[Pt($NH_3$)$_2$-(2-hydroxypyridine)Cl] Y (Y = Cl, $NO_3$) (L.S. Hollis, S.J. Lippard, Inorg. Chem, 22, 2708(1983)), [Pt($NH_3$)$_2$(l-methylcytosine) Cl] X (X = Cl, $NO_3$) (B. Lippert, C.J.L. Lock, R.A. Speranzini, Inorg. Chem, 20, 335(1981), J.F. Britten, B. Lippert, C.J.L. Lock, P. Pilon, Inorg. Chem, 21, 1936(1982)), [Pt(en) (py)Cl]Cl (en = ethylenediamine, py = pyridine (Y.N. Kukuskin, V.P. Kotelnikov, V.N. Spevak, Zh. Obshch.Khim, 43, 2352(1973)), and cis-[Pt($NH_2OH$)$_2$(3-pyridinecarboxamide)Cl]Cl (L.S. Tikhonova, A.I. Stetsenko, Koord.Khim, 9, 2660(1983)). In all of the above cases, no antitumor activity was disclosed.

### SUMMARY OF THE INVENTION

The platinum compounds of this invention are charged Pt(II) complexes containing three neutral amine ligands and one anionic ligand. As such, they violate established structure-activity relationships which state that the requirements for antitumor activity are "a neutral complex with adjacent (cis) reactive X groups" (M.J. Cleare, "Structure-Activity Relationships for Antitumor Platinum Complexes" in D.N. Reinhoudt, T.A. Connors, H.M. Pinedo, K.W. VandePoll, Eds., "Structure-Activity Relationships of Antitumor Agents", Martinus Nijhoff, Boston, 1983). Charged species such as [Pt($NH_3$)$_4$]$Cl_2$ and $K_2PtCl_4$ have been described as completely inactive, and "Triamine" compounds of the type [Pt(dien)X]X (dien = diethylene-triamine, X = Cl,Br) have also shown no antitumor activity (M.J. Cleare and J.D. Hoeschele, Bioinorganic Chem, 2, 187-210 (1973)).

Detailed Description of the Invention

The preferred complexes of the present invention have the following structure:

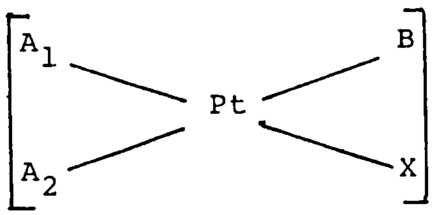

Y (I)

wherein $A_1$ and $A_2$ are ammonia or aliphatic amine ligands, which may or may not be the same and are chosen from ammonia, a linear, cyclic or branched $C_1$-$C_6$ alkylamine, or a linear, cyclic or branched $C_1$-$C_6$ alkylamine, bearing one or more of the following substituents: halo, hydroxy, alkoxy, carboxy, alkoxycarbonyl, or alkylcarboxy.

B is chosen from the following heterocyclic compounds: pyridine, pyrimidine, pyridazine, pyrazine, imidazole, pyrazole, quinoline, isoquinoline or purine. These may be unsubstituted or bear one or more of the following substituents: $C_1$-$C_6$ alkyl, $C_1$-$C_6$ alkoxy, hydroxy, carboxy, $C_1$-$C_6$ alkoxycarbonyl, $C_1$-$C_6$ alkylcarboxy, halo, amine, oxo, nitrite, nitro, or amido.

X is a monodentate anionic ligand such as halo, hydroxo, pseudohalo, $HSO_4$, $H_2PO_4$, ascorbate, or $C_1$-$C_6$ carboxylate.

Y is an anion chosen from X above or nitrate or perchlorate.

X may or may not equal Y.

The platinum(II) triamine complexes of the present invention are prepared by one of two general methods:

$$PtA_1A_2XY + B \longrightarrow [PtA_1A_2BX]Y \qquad (II)$$

$$PtA_1A_2X_2 + AgY \longrightarrow [PtA_1A_2X]Y + Ag\,X$$
$$+$$
$$[PtA_1A_2BX]Y \longleftarrow B \qquad (III)$$

In the method of equation (II), the heterocyclic amine B is reacted directly with a cis-diamineplatinum complex containing two anionic ligands. The reaction is carried out in water or alcohol at a temperature between ambient and the boiling point of the solvent, but preferably, at about 60°C. Each mole of platinum complex is reacted with one or more moles of B, preferably, 1 to 3 moles. The platinum concentration may vary widely but it is usually between 0.01 and 0.1M. The reaction time may vary from several minutes to several days but is usually 1-3 days. Following this reaction period, the mixture may be cooled, if necessary, and the product separated from unreacted starting material and side products by filtration, evaporation to low volume and crystallization. Purification is accomplished by recrystallization from water, water/alcohol, or dilute mineral acid solution or precipitation from solution with a non-solvent. Removal of free B from the product may sometimes be accomplished by washing the product with an organic solvent such as chloroform.

In the method of equation(III) the cis-diamineplatinum complex is first reacted with one equivalent of a silver salt (for example, $AgNO_3$). This reaction may be run in water, alcohol or in a polar organic solvent such as dimethylformamide or dimethylacetamide. The resulting AgX is removed by filtration. The solvated complex is then reacted with one or more moles of B (preferably 1 to 2). The reaction between the cis-diamine platinum complex and silver salt is run at ambient to 80°C, preferably at 50-60°, for a time period of several minutes to 24 hours depending on the solvent system. The mixture is usually cooled to ambient temperature or below prior to filtration of AgX. Reaction of the solvated complex with B may take place at any temperature from 0-80°C, but preferably 25-50°C for a period of several minutes to several days.

The product is separated and purified as in the method of equation(II).

The complexes of this invention are useful in tumor chemotherapy having been found active against

malignant tumors in animals as, for example, Sarcoma 180 ascites tumors in mammals such as mice. The anti-tumor effect exhibited by the subject complexes may also extend to other sarcomas and lymphoid leukemias and to such other tumors as the following: lymphosarcoma, myelocytic leukemia, malignant lymphoma, squamous cell carcinoma, adenocarcinoma, scirrhous carcinoma, malignant melanoma, seminoma, teratoma, choriocarcinoma, embryonalcarcinoma, cystadenocarcinoma, endometriocarcinoma or neuroblastoma and the like. In addition, said complexes may be useful as anti-viral, anti-inflammatory, anti-bacterial and anti-parasitic agents.

The complexes of this invention may be administered parenterally or orally in admixture with a non-toxic pharmacologically acceptable inert carrier or diluent in any of the usual pharmaceutical forms. These include solid and liquid oral unit dosage forms such as tablets, capsules, powders, and suspensions or solutions and suspensions for subcutaneous, intramuscular, intravenous or intra-arterial injection. The term "unit dosage" refers to physically discrete units which may be administered in single or multiple dosages each containing a predetermined quantity of the active ingredient in association with the required diluent, carrier or vehicle. The quantity of active ingredient is the amount of complex needed to produce the desired therapeutic effect.

A typical unit dosage consists essentially of from about 10-450 mg. of active ingredient; however, the form in which said ingredient is administered and the frequency of administration is usually determinative of its concentration. Thus, for example, oral unit dosage forms containing 20-450 mg. of active ingredient may be administered one or more times per day depending upon the severity of the tumor which is sought to be treated and the condition of the host animal. By contrast, parenteral administration generally requires from about 10-100 mg. of the active ingredient per unit dosage administered as a daily dose or as a fraction thereof depending upon whether the regimen calls for administration once, twice, three or four times daily.

By contrast to the "unit dosage", the effective dose is that total dosage which is needed to achieve the desired anti-tumor effect. In general, this dosage lies within the range of from about 10-950 mg. of the active ingredient per kg. of body weight of the host animal (i.e., mg./kg.). A preferred concentration lies within the range of from about 30-450 mg./kg. For oral administration an effective dose of 50-950 mg./kg. has been found to be most suitable, whereas, in the case of parenteral administration it is usually advisable to employ from about 30-350 mg./kg. These dosages are well below the toxic or lethal dose and they may be varied over a wide range for the symptomatic adjustment of the dosage to the patient which is being treated.

The platinum(II)-triamine complexes screened show an effective dose about one-half and in some instances less than one fourth of the toxic dose. This represents a clear improvement over the known complex cis-$[Pt(NH_3)_2Cl_2]$ which, under identical screening, exhibits optimum activity at 9-10 mg./kg. and toxicity at 16 mg./kg. Obviously, the broadest possible range between the effective dose and toxic dose is preferred by clinicians because it affords a wider safety margin.

The preferred compositions for oral administration are tablets in which the triamine complex is present in quantities of 5-375 mg. in a pharmaceutically acceptable orally ingestible solid carrier. If desired, the composition may also contain flavors, binders, lubricants and other excipients known in the art.

An alternative oral mode is the soft gelatin capsule. Such a composition may also contain from 10-375 mg. by weight of active ingredient dissolved or suspended in vegetable oil, peanut oil, alcohol or glycerine and the like.

A hard or dry-filled capsule may be prepared by admixing 25-200 mg. of active ingredient with suitable excipients such as lactose and magnesium stearate and putting the mixture into a preformed and suitably sized gelatin sheath.

The complexes of this invention may also be formulated as liquid solutions or suspensions or as a dry powder for addition to foods, drinking water, fruit juice or other potable liquids.

Tablets are prepared by mixing a complex of this invention, in suitably comminuted or powdered form, with a diluent or base such as starch, sucrose, kaolin or di-calcium phosphate and the like. The resulting mixture can be granulated by wetting with a binder such as a syrup, starch (paste), acacia mucilage or solutions of cellulosic or polymeric materials, whereafter, the wetted mixture is forced through a screen. As an alternative to granulating, the powdered mixture can be run through a tablet machine and any slugs which are imperfect may be broken into granules. The granules are lubricated to prevent sticking to the tablet-forming dies via the addition of stearic acid, a stearate salt, talc or mineral oil and the lubricated mixture is then compressed into tablets. The complexes of this invention can also be combined with free flowing inert carriers and then be subjected to compression so as to form tablets without going through the granulating or slugging steps. A protective coating or sealing coat of shellac, sugar or polymeric material and a polished coating of wax can also be provided. Dyestuffs may be added to distinguish different unit dosages.

In this invention the term "pharmacologically acceptable inert carrier or diluent" denotes a non-toxic substance which, when mixed with the active ingredient, renders it more suitable for administration. Compositions intended for oral administration may include such carriers or diluents as glucose, lactose, sucrose, corn starch, potato starch, sodium carboxymethyl cellulose, ethyl cellulose, cellulose acetate, powdered gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and di-calcium phosphate. Said compositions may also contain non-toxic adjuvants and modifiers such as dyes, buffering agents, preservatives, surfactants, emulsifiers, flavoring agents or biocides and the like.

The following emodiments illustrate the preparation of representative unit dosage forms.

Compressed Tablet

| | |
|---|---|
| Cis-Chlorodiaminepyridineplatinum(II) Chloride | 200 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 380 mg. |

The cis-diamineplatinum(II) pyridine complex, niacinamide, calcium pantothenate, magnesium sulfate, zinc sulfate and magnesium stearate (5.0 Mg.) are mixed and compressed into slugs. The slugs are then broken into granules and sifted through an 8 mesh screen. Additional magnesium stearate (5.0 mg.) is added and the mixture is then compressed into tablets suitable for oral administration.

Soft Gelatin Capsule

A soft elastic gelatin capsule is filled with the following ingredients:

| | |
|---|---|
| Cis-Chlorodiamine-2-hydroxypyridineplatinum(II) Chloride | 100 mg. |
| Wheat germ oil | 50 mg. |
| Sunflower seed oil | 100 mg. |
| | 250 mg. |

The cis-diamineplatinum(II) 2-hydroxy-pyridine complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

Dry Filled Capsule

A hard dry-filled capsule may be prepared from the following ingredients:

| | |
|---|---|
| Cis-Chlorodiamine-6-methyl-2-hydroxypyridineplatinum(II) Chloride | 200 mg. |
| Niacinamide | 50 mg. |
| Calcium Pantothenate | 10 mg. |
| Sodium Ascorbate | 150 mg. |
| | 410 mg. |

The cis-diamineplatinum(II) 6-methyl-2-hydroxypyridine complex is reduced to a No. 60 powder. Niacinamide, calcium pantothenate and sodium ascorbate are passed through a No. 60 bolting cloth and these ingredients are added to the cis-diamineplatinum(II) pyridine complex. This combination of ingredients is mixed for 10 minutes and then poured into a No. !3 size gelatin capsule.

Dry Powder

The following composition illustrates a representative dosage in dry powder form. In this embodiment the active ingredient is water soluble and it is combined with up to 60% by weight of a suitable flavoring agent. All quantities are in a weight-percent relationship.

| | |
|---|---|
| Cis-Chlorodiamine-3-hydroxypyridineplatinum(II) Chloride | 25-90 |
| Flavoring Agent | 10-60 |
| Preservative | 0.1% |

The cis-diamineplatinum(II) 3-hydroxy-pyridine complex, flavoring agent and preservative are thoroughly blended to afford a homogeneous dry powder which is readily soluble in water. The resulting formulation may be used as a food additive or it may be blended with other therapeutic agents to afford combination-type medicinals. Alternatively, said powder may be dissolved in a pharmacologically acceptable diluent to afford a solution which is suitable for oral administration.

Compositions intended for parenteral administration may include such diluents and carriers as water-miscible solvents as, for example, sesame oil, groundnut oil, aqueous propylene glycol and a solution of sodium riboflavin. Typical of said compositions are solutions which contain the active ingredient in sterile form. An embodiment illustrating a dosage form suitable for intravenous injection is set forth below.

Parenteral Solution

Injectable solutions can be formulated by mixing an ampoule of active ingredient with an ampoule of sterile diluent:

| | | |
|---|---|---|
| Ampoule: | Cis-Chlorodiammine pyridineplatinum(II)chloride | 300 mg. |
| Ampoule: | Sterile water (Diluent for Injection) | 4 cc. |

The cis-diamminepyridine(II)chloride complex and water are mixed thorougly immediately prior to administration. If desired, one or more other active ingredients may be added to provide an injectable solution having enhanced therapeutic activity.

Examples 1-18, infra, illustrate the methods by which the compounds of this invention may be obtained and Example 21 describes the protocol used to evaluate their efficacy in mice. However, said examples are illustrative only and thus inventions should not be construed as being limited thereto because it will be apparent to one of ordinary skill that modifications may be effected and reagents may be substituted for those recited therein without departing from the scope of this invention as defined in the appendant claims.

EXAMPLE 1

Cis-Chlorodiamminepyridineplatinum(II) Chloride, cis-$[Pt(NH_3)_2(py)Cl]Cl$

Cis-$[Pt(NH_3)_2Cl_2]$ (6.0g, 20 mmole) and pyridine (1.6ml, 20 mmoles) were added to 1 L $H_2O$ and heated to 60° C with stirring for 2 hours. Following this, the reaction mixture was cooled to room temperature and allowed to stir for 72 hours. The volume was then reduced to ca. 100ml, at which time unreacted cis-(Pt-$(NH_3)_2Cl_2]$ was removed by filtration. The filtrate was taken to dryness and the resulting yellowish white solid was recrystallized once from 0.1N HCl, once from 0.5N HCl, and once from methanol. The yield was 1.92g (25.3%) of white solid. Anal. calc for $PtC_5H_{11}N_3Cl_2$:Pt, 51.45; C, 15.84; H, 2.92; N, 11.08; Cl, 18.70. Found: Pt, 51.60; C, 15.67; H, 2.85; N, 10.98:; Cl, 18.98. The infrared spectrum of Cis-$[Pt(NH_3)_2(py)Cl]Cl$ is given in Figure 1.

EXAMPLE 2

Cis-Chlorodiammine-2-hydroxypyridineplatinum(II) Chloride, cis-$[Pt(NH_3)_2(2\text{-}pyOH)Cl]Cl$

Cis-$[Pt(NH_3)_2Cl_2]$ (15.0 g, 50 mmole) and 2-hydroxypyridine (10.7g, 112.5 mmole) was stirred in 1.35 L $H_2O$ at 60° C for 48 hours. The resulting solution was cooled and evaporated to ca. 150 ml and filtered to

remove a greenish solid. The solution was then evaporated to dryness and the residue stirred in ca. 500 ml $CH_2Cl_2$ to remove unreacted 2-pyOH. Following filtration and air drying, 2.5 g of the crude product was dissolved in 100 ml of methanol, and a small amount of yellow solid removed by filtration. The filtrate was evaporated to dryness and solid recrystallized from 0.1N HCl (4.5ml) yielding 1.02g white crystals after air drying. Based on this, a projected yield of 6.45g (32.6%) would be obtained from the entire preparation. The infrared spectrum of cis-[Pt($NH_3$)$_2$-(2-pyOH)Cl]Cl is given in Figure 2.

EXAMPLE 3

Cis-Chlorodiammine-6-methyl-2-hydroxypyridineplatinum(II) Chloride cis-[Pt($NH_3$)$_2$(6-Me-2-pyOH)Cl]Cl

Cis-[Pt($NH_3$)$_2$$Cl_2$] (10.0g, 33.3 mmoles) and 6-methyl-2-hydroxypyridine (5.0g, 45.8 mmoles) were stirred in 900 ml $H_2O$ at ca. 60 °C for 48 hrs. A dark brown solid was filtered form the reaction mixture and the filtrate evaporated to ca. 450 ml. The yellow precipitate which had formed was removed by filtration and evaporation continued to dryness. The residue was treated with methanol (ca. 500ml) and the insoluble portion removed by filtration. The filtrate was taken to dryness and the resulting solid treated with $CHCl_3$ -(100ml). The chloroform insoluble portion was collected and air dried yielding 6.4g khaki green solid. One gram of this material was recrystallized from methanol/dilute HCl yielding 0.42g amber crystals. For the entire preparation the projected yield is 2.69g (19.7%). Anal. Calc for $PtC_6H_{13}N_3OCl_2$:Pt, 47.68; C, 17.61; H, 3.20; N, 10.27;Cl, 17.33. Found: Pt, 47.43; C, 17.64; H, 3.22; N, 10.13; Cl, 17.33. The infrared spectrum of cis-[Pt($NH_3$)$_2$(6-Me-2-py-OH)Cl]Cl is shown in Figure 3.

EXAMPLE 4

Cis-chlorodiammine-3-hydroxypyridineplatinum(II) Chloride cis-[Pt($NH_3$)$_2$(3-pyOH)Cl]Cl

Cis-[Pt($NH_3$)$_2$$Cl_2$] (10.0 g, 33.3 mmoles) and 3-hydroxypyridine (7.2g, 75.7 mmoles) were stirred in 900ml $H_2O$ at 60 °C for 48 hours, resulting in a clear solution. This was evaporated to ca. 450 ml, and the yellow solid which had formed was removed by filtration. The filtrate was taken to dryness and dissolved in methanol (25ml). The insoluble portion was removed by filtration and the methanol solution evaporated to dryness. A 2.0g portion of the remaining solid was recrystallized from hot 0.1N HCl (4.0ml) yielding 1.30g of white crystals. Anal. Calc for $PtC_5H_{11}N_3OCl_2$; Pt, 49.37; C, 15.20; H, 2.81; N, 10.63; Cl, 17.94. Found: Pt, 49.50; C, 15.38; H, 2.83; N, 10.50; Cl, 17.71. The infrared spectrum of cis-[Pt($NH_3$)$_2$(3-pyOH)Cl]Cl is given in Figure 4.

EXAMPLE 5

Preparation of Cis-chlorodiammine-4-methylpyridineplatinum(II)chloride, cis-[Pt($NH_3$)$_2$(4-$CH_3$-py)Cl]Cl

Cis-[Pt($NH_3$)$_2$$Cl_2$] (6.0g, 20 mmoles) and 4-methylpyridine (1.95 ml, 20 mmoles) were stirred at 50-60 °C for 36 hours. The volume of the solution was reduced under vacuum to ca. 50 ml, with insoluble material removed several times in the course of the evaporation. The filtrate was taken to dryness and the residue dissolved in methanol (120 ml). The insoluble portion was removed by filtration and the filtrate evaporated to dryness. This residue was treated with ethanol (20 ml), the insolubles removed by filtration, and the filtrate evaporated to dryness, leaving 2.1 g of light yellow crystals. The infrared spectrum of cis-[Pt($NH_3$)$_2$(4-$CH_3$-py)Cl]Cl is shown in Figure 5.

EXAMPLE 6

Cis-chlorodiamminecytosineplatinum(II) chloride, [Pt($NH_3$)$_2$(cytosine)Cl]Cl

Cisplatin (6.00g, 20 mmoles) and cytosine (2.3g, 20 mmoles) were stirred in 1 liter $H_2O$ for 2 days at 60 °C. After cooling to room temperature and removing insolubles by filtration, the solution was evaporated on a rotary evaporator to ca. 30 ml and refrigerated. After 2 hours, a light purple solid precipitated from the solution and was collected by filtration. This was recrystallized from 0.5 M HCl (15 ml) yielding two crops (total weight 2.05g) of purple tinged crystals. Recrystallization of a portion of this from water yielded colorless crystals. Anal. calc for $PtC_4H_{11}N_5Cl_2O$: Pt, 47.45; C, 11.68; H, 2.70; N, 17.03; Cl, 17.25. Found: Pt, 47.35; C, 11.64; H, 2.77; N, 17.00; Cl, 17.42. The infrared spectrum of cis-[Pt($NH_3$)$_2$(cytosine)Cl]Cl is given

in Figure 6.

EXAMPLE 7

Cis-chlorodiammine-6-methyl-3-hydroxypyridineplatinum(II) chloride, cis-[Pt($NH_3$)$_2$(6-Me-3-pyOH)Cl]Cl

To 1 liter of water was added cisplatin (6.0g, 20 mmoles and 6-methyl-3-hydroxypyridine (2.4g, 26 mmoles), and the mixture heated at 55°C for 3 days. The resulting solution was cooled to room temperature and evaporated on a rotary evaporator to ca. 35 ml. This was mixed with ca. 100 ml methanol and filtered to remove unreacted cisplatin. The filtrate was taken to 30 ml and then allowed to further evaporate in the hood. The resulting solid was filtered and recrystallized from a small volume of methanol yielding colorless crystals (1.37g). Anal. calc. for $PtC_6H_{13}N_3Cl_2O$: Pt, 45.67; C, 16.87; H, 3.54; N, 9.84; Cl, 16.60. Found: Pt, 44.31; C, 17.00; H, 3.38; N, 9.86; Cl, 16.88. The infrared spectrum of cis-[Pt($NH_3$)$_2$ H, 3.54; (6-Me-3-pyOH )Cl]Cl is given in Figure 7.

EXAMPLE 8

Cis-chlorodiammine-4-chloropyridineplatinum(II) chloride, cis-[Pt($NH_3$)$_2$(4-Cl-py)Cl]Cl

Cisplatin (6.0g, 20 mmoles) and 4-chloropyridine (3.1g, 20 mmoles) were added to 1 liter of water and the mixture stirred at 60° for 48 hrs. On cooling, the resulting mixture was alternately filtered and evaporated on a rotary evaporator until all of the unreacted cisplatin was removed. The filtrate was then taken to dryness leaving a cream colored residue which was recrystallized from hot methanol, yielding 2.05g product. Anal. calc. for $PtC_5H_{10}N_3Cl_3$: Pt, 47.17; C, 14.52; H, 2.44; N, 10.16; Cl, 25.72. Found: Pt, 46.57, C, 14.54; H, 2.54, N, 10.16; Cl 25.28. The infrared spectrum of cis-[Pt($NH_3$)$_2$(4-Cl-py)Cl]Cl is given in Figure 8.

EXAMPLES 9-17

The following complexes can also be prepared using the methods of Examples 1-8.

| Example | $A_1$ | $A_2$ | B | X | Y |
|---|---|---|---|---|---|
| 9 | $CH_3NH_2$ | $CH_3NH_2$ | pyridine | Cl | $NO_3$ |
| 10 | $NH_3$ | $NH_3$ | guanine | Br | Br |
| 11 | $(HOCH_2)_3CNH_2$ | $(HOCH_2)_3CNH_2$ | pyrazole | Cl | Cl |
| 12 | $NH_3$ | $(CH_3)_2NH$ | imidazole | Cl | Cl |
| 13 | $i\text{-}C_3H_7NH_2$ | $i\text{-}C_3H_7NH_2$ | 4-pyridine-carboxylic acid | Cl | $NO_3$ |
| 14 | 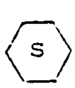 $-NH_2$ | 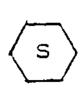 $-NH_2$ | imidazole | I | $HSO_4$ |
| 15 | $NH_3$ | $HOCH_2CH_2NH_2$ | pyrazine | Cl | Cl |
| 16 | $C_2H_5NH_2$ | $C_2H_5NH_2$ | uracil | Br | $NO_3$ |
| 17 | $NH_3$ | $NH_3$ | 4-cyano-pyridine | $H_2PO_4$ | $NO_3$ |

The following compounds do not show antitmor activity:

EXAMPLE 18 (Comparative)

Trans-Chlorodiamminepyridineplatinum(II) Chloride, trans-[Pt($NH_3$)$_2$(py)Cl]Cl

Trans-Pt($NH_3$)$_2$$Cl_2$ (2.05g, 6.83 mmoles) and pyridine (0.66ml, 8.15 mmoles) were stirred in 500ml of water at ca. 60°C for 24 hours. The resulting solution was evaporated to dryness under vacuum. The residue was transferred to another container using methanol, and the methanol allowed to evaporate. The remaining solid was dissolved in 30mL water by heating on a steam bath. On cooling to ca. 5° for 1 hour a white solid was deposited, which was collected by filtration and washed with a small quantity of cold water, then with methanol. After air drying, 1.3 g (50% yield) of white solid remained. Anal. calc for $PtC_5H_{11}N_3Cl_2$: Pt, 51.45; C, 15.84; H, 2.92; N, 11.08; Cl, 18.70. Found: Pt, 51.20; C, 15.67; H, 3.11; N, 10.96; Cl, 18.83. The infrared spectrum of trans-[Pt($NH_3$)$_2$(py)Cl]Cl is given in Figure 10.

EXAMPLE 19 (Comparative)

Chloro-1,2-diaminocyclohexanepyridineplatinum(II) chloride, [Pt(dach)(py)Cl]Cl

Pt(dach)$Cl_2$ (3.802g, 10 mmoles) and $AgNO_3$ (1.698g, 10 mmoles) were mixed in dimethylformamide (100ml) and stirred for 2 hours at room temperature. The AgCl was removed by filtration, and pyridine (0.8g, 10.1 mmoles) was added to the reaction mixture, which was then stirred for 18 hours. Additional AgCl, which had formed after the pyridine was added, was removed by filtration. The filtrate was poured into ca. 300 ml ether, resulting in a precipitate which turned into an oil after stirring for 15 minutes. The ether was decanted and the oil was triturated with isopropanol. The isopropanol filtrate was evaporated to dryness, yielding a solid which was washed with ethanol. The solid was recrystallized from methanol to yield 0.6g white solid. Anal. Calc for $PtC_{11}H_{19}N_3Cl_2$: Pt, 42.47; C, 28.76; H, 4.17; N, 9.15; Cl, 15.44. Found: Pt, 42.18; C, 28.71; H, 4.27; N, 9.16; Cl, 15.40.

EXAMPLE 20

Screening of Compounds for Anti-Tumor Activity Against S180a

The compounds were evaluated for anti-tumor activity against S180 ascites in female CFW Swiss mice by the following procedure:

CFW mice, averaging 20g, are immediately inspected and placed in newly prepared cages. On day zero the mice are inoculated with 0.2 ml. of a freshly prepared saline suspension (0.15 M NaCl) containing 1 x $10^7$ tumor cells/ml, or a total of 2 x $10^6$ cells. This inoculum is freshly prepared using "transfer" mice which have been injected with tumor cells the previous week. This inoculum is the end-product of a series of steps which involves (1) the removal of the cells from the peritoneal cavity of the sacrificed transfer mouse, (2) alternate centrifugation-washing (2-3 times with cold saline) to remove occasional blood and other undesirable components, and finally (3) dilution (1:3) of the packed cell volume with saline (the final centrifugation being carried out at 1,000 rpm for 2 min.). A cell count is made on a 2,000-fold dilution of this 1:3 suspension by means of a Coulter Counter. A final dilution to 1 x $10^7$ cells/ml. is made based on the averaged count. On day 1, solutions of the test compounds are prepared and the mice injected, with each mouse of a set of six being injected with the same test compound at the same dose level.

Also, on this day, two types of controls (6 mice/set are employed: (1) Normal (1 set): 0.5 ml. of the solvent medium used for the test compound, and (2) Positive control (1 set): a known anti-tumor agent (cis-[Pt($NH_3$)$_2$$Cl_2$] in saline at 8 mg./kg.), used to test the response of the biological system.

The effectiveness of a compound is measured in terms of the increase in life span (ILS) of the test animals relative to the controls (calculated from the day of tumor inoculation (day zero). In order to standardize the test data and permit intercomparisons to be made, the day of evaluation is arbitrarily taken as that day corresponding to twice the mean life-span (or average day of death) of the normal controls. This sets a practical upper limit of 100% on the ILS attainable. For calculation purposes, survivors on the day of evaluation are considered to have died on that day. The % ILS is formulated as:

$$\%\ ILS = \left( \frac{\text{mean life-span of test mice}}{\text{mean life-span of control mice}} - 1 \right) \times 100\%$$

ILS values above 50% represent significant activity; those above 75% represent excellent activity.

Sarcoma 180 ascites antitumor screening data for the platinum-pyridine complexes described in

Examples 1-9 and 19-20 are given in Table 1. The compounds of Examples 1-9 met the criterion for activity (at least 50% ILS at one or more doses). Cis-[Pt($NH_3$)$_2$(py)Cl]Cl gave the highest level of activity, 100% ILS at 40 mg/kg (the maximum possible under the screening protocol). The compounds were tested in water in all cases, and were completely soluble at all of the dose levels employed.

TABLE 1

S180a ANTITUMOR SCREENING DATA

| Compound (Code) | Medium | Dose mg/kg | %ILS | 30-Day Survivors | Positive Control[a] %ILS | Positive Control[a] 30-Day Survivors |
|---|---|---|---|---|---|---|
| cis-[Pt($NH_3$)$_2$(py)Cl]Cl Example 1 | $H_2O$ | 10 | 49 | 0/6 | 80 | 2/7 |
| | | 20 | 70 | 0/6 | | |
| | | 40 | 100 | 6/6 | | |
| | | 80 | 67 | 4/6 | | |
| | | 160 | -74 | 0/6 | | |
| | | 320 | -94 | 0/7 | | |
| cis-[Pt($NH_3$)$_2$(py)Cl]Cl Example 1 | $H_2O$ | 5 | 19 | 0/6 | 79 | 2/6 |
| | | 10 | 15 | 0/6 | | |
| | | 20 | 73 | 1/6 | | |
| | | 40 | 91 | 4/6 | | |
| | | 80 | -27 | 0/6 | | |
| | | 160 | -88 | 0/6 | | |
| cis-[Pt($NH_3$)$_2$(2-pyOH)Cl]Cl Example 2 | $H_2O$ | 40 | 95 | 4/6 | 79 | 4/6 |
| | | 80 | 91 | 4/6 | | |
| | | 160 | 64 | 3/6 | | |
| | | 320 | -74 | 0/6 | | |
| | | 640 | -77 | 0/6 | | |
| | | 1280 | -84 | 0/6 | | |
| cis-[Pt($NH_3$)$_2$(2-pyOH)Cl]Cl Example 2 | $H_2O$ | 5 | 15 | 0/6 | 66 | 1/6 |
| | | 10 | 15 | 0/6 | | |
| | | 20 | 28 | 0/6 | | |
| | | 40 | 78 | 2/6 | | |
| | | 80 | 80 | 2/6 | | |
| cis-[Pt($NH_3$)$_2$(6-Me-2-pyOH)Cl]Cl Example 3 | $H_2O$ | 10 | -9 | 0/6 | 79 | 3/6 |
| | | .0 | -[illegible] | 0/6 | | |
| | | 40 | 12 | 0/6 | | |
| | | 80 | 15 | 0/6 | | |
| | | 160 | 93 | 5/6 | | |
| | | 320 | 93 | 3/6 | | |

TABLE 1 (Continued)

S180a ANTITUMOR SCREENING DATA

| Compound (Code) | Medium | Dose mg/kg | %ILS | 30-Day Survivors | Positive Control %ILS | Positive Control 30-Day Survivors |
|---|---|---|---|---|---|---|
| cis-[Pt($NH_3$)$_2$(3-pyOH)Cl]Cl Example 4 | $H_2O$ | 10 | 5 | 0/6 | 79 | 3/6 |
| | | 20 | 10 | 0/6 | | |
| | | 40 | 54 | 0/6 | | |
| | | 80 | 69 | 1/6 | | |
| | | 160 | - 8 | 1/6 | | |
| | | 320 | -75 | 0/6 | | |
| cis-[Pt($NH_3$)$_2$(4-$CH_3$-py)Cl]Cl Example 5 | $H_2O$ | 10 | 5 | 0/6 | 79 | 3/6 |
| | | 20 | 27 | 1/6 | | |
| | | 40 | 83 | 2/6 | | |
| | | 80 | 66 | 1/6 | | |
| | | 160 | -77 | 0/6 | | |
| | | 320 | -91 | 0/6 | | |
| cis-[Pt($NH_3$)$_2$(cytosine)Cl]Cl Example 6 | $H_2O$ | 20 | 23 | 0/6 | 94 | 4/6 |
| | | 40 | 58 | 1/6 | | |
| | | 80 | 92 | 4/6 | | |
| | | 160 | 68 | 3/6 | | |
| | | 320 | -16 | 2/6 | | |
| | | 640 | -74 | 0/6 | | |
| cis-[Pt($NH_3$)$_2$(6-Me-3-pyOH)Cl]Cl Example 7 | $H_2O$ | 20 | 30 | 0/6 | 73 | 1/6 |
| | | 40 | 70 | 2/6 | | |
| | | 80 | 93 | 4/6 | | |
| | | 160 | 82 | 3/6 | | |
| | | 320 | -49 | 0/6 | | |
| | | 640 | -75 | 0/6 | | |

TABLE 1 (Continued)

S180a ANTITUMOR SCREENING DATA

| Compound (Code) | Medium | Dose mg/kg | %ILS | 30-Day Survivors | Positive Control[a] %ILS | Positive Control[a] 30-Day Survivors |
|---|---|---|---|---|---|---|
| cis-[Pt$(NH_3)_2$(4-Cl-py)Cl]Cl | $H_2O$ | 10 | 22 | 0/6 | 72 | 3/6 |
| Example 8 | | 20 | 68 | 2/6 | | |
| | | 40 | 77 | 4/6 | | |
| | | 80 | 36 | 4/6 | | |
| | | 160 | -77 | 0/6 | | |
| | | 320 | -94 | 0/6 | | |
| trans-[Pt$(NH_3)_2$(py)Cl]Cl | $H_2O$ | 5 | 2 | 0/6 | 98 | 3/6 |
| Example ~~19~~ 18 (Comparative) | | 10 | 7 | 0/6 | | |
| | | 20 | 6 | 0/6 | | |
| | | 40 | 15 | 0/6 | | |
| | | 80 | 26 | 0/6 | | |
| | | 160 | -70 | 0/6 | | |
| [Pt(dach)(py)Cl]Cl | $H_2O$ | 10 | 6 | 0/6 | 73 | 1/6 |
| Example ~~20~~ 19 (Comparative) | | 20 | 8 | 0/6 | | |
| | | 40 | 6 | 0/6 | | |
| | | 80 | 15 | 0/6 | | |
| | | 160 | 25 | 0/6 | | |
| | | 320 | -94 | 0/6 | | |

[a] Positive control = 8 mg/kg cis-[Pt$(NH_3)_2Cl_2$]

## Claims

**1.** A cis-triamine platinum (II) complex having the general formula

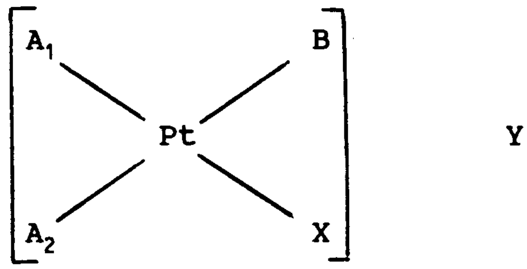

wherein Pt is in a valence state II and is coordinated to $A_1$ and $A_2$ in a cis configuration, wherein $A_1$ and $A_2$ may or may not be the same and are chosen from ammonia or a monodentate linear, cyclic or branched $C_1$-$C_6$ alkylamine, or a linear, cyclic or branched $C_1$-$C_6$ alkylamine bearing one or more of the following substitutents: halo, hydroxy, alkoxy, carboxy, alkoxycarbonyl or alkylcarboxy, B is a substituted pyridine selected from the group consisting of 3-hydroxypyridine, 6-methyl-2-hydroxypyridine, 6-methyl-3-hydroxypyridine, 4-methyl-pyridine and 4-chloropyridine, X is a monodentate anionic ligand, such as halo, hydroxo, pseudohalo, $HSO_4$, $H_2PO_4$, ascorbate or $C_1$-$C_6$ carboxylate, and Y is an anion chosen from X above or nitrate or perchlorate.

**2.** The complex according to claim 1 wherein $A_1$ and $A_2$ are ammonia or alkylamine having up to 6 carbons in the alkyl group.

**3.** The complex according to claim 2 wherein $A_1$ and $A_2$ are ammonia.

**4.** The complex according to any of claims 1 to 3 wherein both X and Y are halides.

**5.** The complex according to claim 4 wherein said halide in chlorine.

**6.** The complex according to any of claims 1 to 5 wherein B is 3-hydroxypyridine.

**7.** The complex according to any of claims 1 to 5 wherein B is 6-methyl-2-hydroxypyridine.

**8.** The complex according to any of claims 1 to 5 wherein B is 6-methyl-3-hydroxypyridine.

**9.** The complex according to any of claims 1 to 5 wherein B is 4-methylpyridine.

**10.** The complex according to any of claims 1 to 5 wherein B is 4-chloropyridine.

**11.** A pharmaceutical composition comprising an active ingredient mixed with a nontoxic pharmaceutically inert carrier, said active ingredient comprising a triamine platinum (II) complex having the formula:

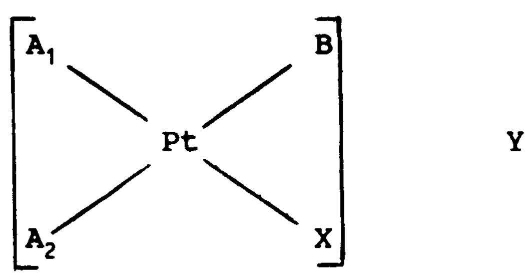

wherein Pt is in a valence state II and is coordinated to $A_1$ and $A_2$ in a cis configuration, wherein $A_1$ and $A_2$ may or may not be the same and are chosen from ammonia or a monodentate linear, cyclic or branched $C_1$-$C_6$ alkylamine, or a linear, cyclic or branched $C_1$-$C_6$ alkylamine bearing one or more of the following substitutents: halo, hydroxy, alkoxy, carboxy, alkoxycarbonyl or alkylcarboxy, B is a N-containing heterocyclic or substituted heterocyclic compound chosen from the group consisting of pyridine, pyrimidine, pyridazine, pyrazine, imidazole, pyrazole, quinoline, isoquinoline or purine, or any

of the foregoing bearing one or more of the following substitutents: $C_1$-$C_6$ alkyl, $C_1$-$C_6$alkoxy, hydroxy, carboxy, $C_1$-$C_6$ alkoxycarbonyl, $C_1$-$C_6$ alkylcarboxy, halo, amine, oxo, nitrite, nitro or amido, X is a monodentate anionic ligand such as halo, hydroxo, pseudohalo, $HSO_4$, $H_2PO_4$, ascorbate or $C_1$-$C_6$ carboxylate, and Y is an anion chosen from X above or nitrate or perchlorate.

**12.** The composition of claim 11 wherein B is pyridine.

**13.** The composition of claim 11 wherein B is a substituted pyridine.

**14.** The composition of claim 13 wherein B is 3-hydroxypyridine.

**15.** The composition of claim 13 wherein B is 6-methyl-2-hydroxypyridine.

**16.** The composition of claim 13 wherein B is 6-methyl-3-hydroxypyridine.

**17.** The composition of claim 13 wherein B is 4-methylpyridine.

**18.** The composition of claim 13 wherein B is 4-chloropyridine.

**19.** The composition of claim 13 wherein B is cytosine.

**20.** The composition of claim 13 wherein B is 2-hydroxypyridine.

**21.** The composition of any of claims 11 to 20 wherein X and Y are chlorine.

**22.** The composition of any of claims 11 to 21, adapted for parenteral administration.

**23.** The composition of any of claims 11 to 21, adapted for oral administration.

**24.** The composition of any of claims 11 to 23, adapted for administration in a single dose.

**25.** The composition of any of claims 11 to 23, adapted for administration in multiple doses.

**26.** A triamine platinum complex of the general formula:

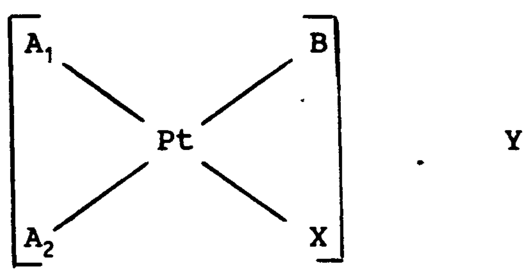

wherein Pt is in a valence state II and is coordinated to $A_1$ and $A_2$ in a cis configuration, and wherein $A_1$, $A_2$, B, X and Y are as defined in any of claims 11 to 20, for use in therapy.

**27.** The use of a triamine platinum complex of the general formula set forth in claim 26 in the manufacture of a medicament for treating animal malignant tumor cells.

**28.** A method of making a platinum complex according to claim 1, comprising reacting a compound of the general formula

$PtA_1A_2XY$

with a compound of the general formula B, wherein Pt is in a valence state II and is coordinated to $A_1$ and $A_2$ in a cis configuration, and wherein $A_1$, $A_2$, X, Y and B are as defined in any of claims 1 to 10.

**29.** A method according to claim 28, wherein said compound of general formula

$PtA_1A_2XY$

is prepared by reacting a compound of the general formula

$PtA_1A_2X_2$

with a silver salt of general formula AgY.

**30.** A method of making a composition according to any of claims 11 to 25, comprising mixing said nontoxic pharmaceutically inert carrier with said active ingredient.

**Revendications**

**1.** Complexe de platine (II) cis-triamine ayant la formule générale

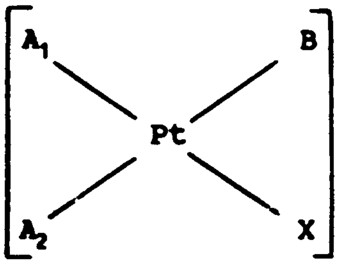

Y

où Pt est dans un état de valence II et est coordiné à $A_1$ et $A_2$ dans une configuration cis, $A_1$ et $A_2$ peuvent être identiques ou différents et sont choisis parmi l'ammoniac ou une alkyl($C_1$-$C_6$)amine monodentée linéaire, cyclique ou ramifiée, ou une alkyl($C_1$-$C_6$)amine linéaire, cyclique ou ramifiée portant un ou plusieurs des substituants suivants: halogéno, hydroxy, alcoxy, carboxy, alcoxycarbonyle ou alkylcarboxy, B est une pyridine substituée choisie parmi la 3-hydroxypyridine, la 6-méthyl-2-hydroxypyridine, la 6-méthyl-3-hydroxypyridine, la 4-méthyl-pyridine et la 4-chloropyridine, X est un ligand anionique monodenté, tel que halogéno, hydroxo, pseudohalogéno, $HSO_4$, $H_2PO_4$, ascorbate ou carboxylate en $C_1$-$C_6$, et Y est un anion choisi parmi X susdit ou nitrate ou perchlorate.

**2.** Complexe selon la revendication 1, dans lequel $A_1$ et $A_2$ sont l'ammoniac ou une alkylamine ayant jusqu'à 6 atomes de carbone dans le groupe alkyle.

**3.** Complexe selon la revendication 2, dans lequel $A_1$ et $A_2$ sont l'ammoniac.

**4.** Complexe selon l'une quelconque des revendications 1 à 3, dans lequel X et Y sont tous deux des halogénures.

**5.** Complexe selon la revendication 4, dans lequel ledit halogénure est du chlore.

**6.** Complexe selon l'une quelconque des revendications 1 à 5, dans lequel B est la 3-hydroxypyridine.

**7.** Complexe selon l'une quelconque des revendications 1 à 5, dans lequel B est la 6-méthyl-2-hydroxypyridine.

**8.** Complexe selon l'une quelconque des revendications 1 à 5, dans lequel B est la 6-méthyl-3-hydroxypyridine.

**9.** Complexe selon l'une quelconque des revendications 1 à 5, dans lequel B est la 4-méthylpyridine.

**10.** Complexe selon l'une quelconque des recendications 1 à 5, dans lequel B est la 4-chloropyridine.

**11.** Composition pharmaceutique comprenant un ingrédient actif mélangé avec un support pharmaceutiquement inerte non-toxique, ledit ingrédient actif comprenant un complexe de platine (II) triamine ayant la formule:

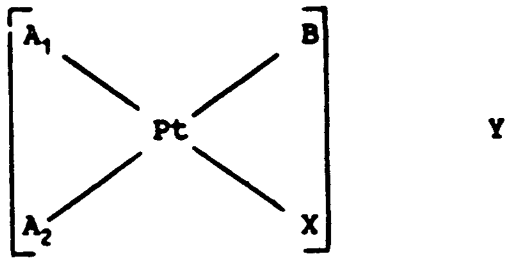

où Pt est dans un état de valence II et est coordiné à $A_1$ et $A_2$ dans une configuration cis, $A_1$ et $A_2$ peuvent être identiques ou différents et sont choisis parmi l'ammoniac ou une alkyl($C_1$-$C_6$)amine monodentée linéaire, cyclique ou ramifiée, ou une alkyl($C_1$-$C_6$)amine linéaire, cyclique ou ramifiée portant un ou plusieurs des substituants suivants: halogéno, hydroxy, alcoxy, carboxy, alcoxycarbonyle ou alkylcarboxy, B est un composé hétérocyclique ou hétérocyclique substitué contenant de l'azote choisi parmi la pyridine, la pyrimidine, la pyridazine, la pyrazine, l'imidazole, le pyrazole, la quinoléine, l'isoquinoléine et la purine, ou l'une quelconque de celles-ci portant un ou plusieurs des substituants suivants: alkyle en $C_1$-$C_6$, alcoxy en $C_1$-$C_6$, hydroxy, carboxy, alcoxy($C_1$-$C_6$)carbonyle, alkyl($C_1$-$C_6$)-carboxy, halogéno, amine, oxo, nitrite, nitro ou amido, X est un ligant anionique monodenté tel que halogéno, hydroxo, pseudohalogéno, $HSO_4$, $H_2PO_4$, ascorbate ou carboxylate en $C_1$-$C_6$, et Y est un anion choisi parmi X susdit ou nitrate ou perchlorate.

**12.** Composition selon la revendication 11, dans laquelle B est la pyridine.

**13.** Composition selon la revendication 11, dans laquelle B est une pyridine substituée.

**14.** Composition selon la revendication 13, dans laquelle B est la 3-hydroxypyridine.

**15.** Composition selon la revendication 13, dans laquelle B est la 6-méthyl-2-hydroxypyridine.

**16.** Composition selon la revendication 13, dans laquelle B est la 6-méthyl-3-hydroxypyridine.

**17.** Composition selon la revendication 13, dans laquelle B est la 4-méthylpyridine.

**18.** Composition selon la revendication 13, dans laquelle B est la 4-chloropyridine.

**19.** Composition selon la revendication 13, dans laquelle B est la cytosine.

**20.** Composition selon la revendication 13, dans laquelle B est la 2-hydroxypyridine.

**21.** Composition selon l'une quelconque des revendications 11 à 20, dans laquelle X et Y sont le chlore.

**22.** Composition selon l'une quelconque des revendications 11 à 21, adaptée pour une administration parentérale.

**23.** Composition selon l'une quelconque des revendications 11 à 21, adaptée pour une administration orale.

**24.** Composition selon l'une quelconque des revendications 11 à 23, adaptée pour une administration en une dose unitaire.

**25.** Composition selon l'une quelconque des revendications 11 à 23, adaptée pour une administration en doses multiples.

**26.** Complexe de platine triamine de formule générale:

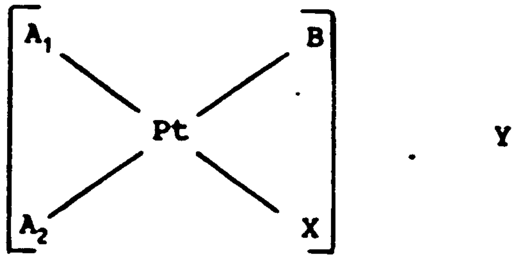

où Pt est dans un état de valence II et est coordiné à $A_1$ et $A_2$ dans une configuration cis, et où $A_1$, $A_2$, B, X et Y sont tels que définis dans l'une quelconque des revendications 11 à 20, pour une utilisation en thérapie.

**27.** Utilisation d'un complexe de platine triamine de formule générale selon la revendication 26 dans la production d'un médicament pour le traitement des cellules tumorales malignes d'animaux.

**28.** Procédé pour la production d'un complexe de platine selon la revendication 1, comprenant la réaction d'un composé de formule générale

$PtA_1A_2XY$

avec un composé de formule générale B, Pt étant dans un état de valence II et étant coordiné à $A_1$ et $A_2$ dans une configuration cis, et $A_1$, $A_2$, X, Y et B étant tels que définis dans l'une quelconque des revendications 1 à 10.

**29.** Procédé selon la revendication 28, dans lequel ledit composé de formule générale

$PtA_1A_2XY$

est préparé par réaction d'un composé de formule générale

$PtA_1A_2X_2$

avec un sel d'argent de formule générale AgY.

**30.** Procédé pour la production d'une composition selon l'une quelconque des revendications 11 à 25, comprenant le mélange du dit support pharmaceutiquement inerte non-toxique avec ledit ingrédient actif.

**Patentansprüche**

**1.** Cis-Triaminplatin(II)-Komplex mit der allgemeinen Formel

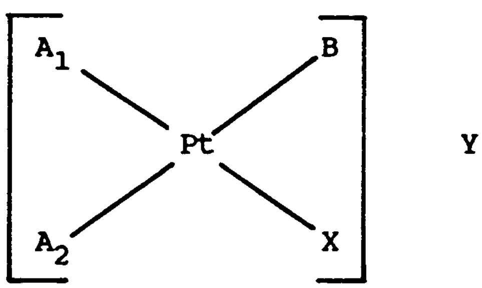

worin Pt in einem Wertigkeitszustand II ist und mit $A_1$ und $A_2$ in einer cis-Konfiguration koordiniert ist,

worin $A_1$ und $A_2$ gleich oder verschieden sein können und aus Ammoniak oder einem linearen, cyklischen oder verzweigten $C_1$-$C_6$ Alkylamin-Monodentat oder einem linearen, cyklischen oder verzweigten $C_1$-$C_6$ Alkylamin-Monodentat, das ein oder mehrere der folgenden Substituenten Halogen, Hydroxy, Alkoxy, Carboxy, Alkoxycarbonyl oder Alkylcarboxy trägt, gewählt werden,

B ein substituiertes Pyridin, gewählt aus der Gruppe, bestehend aus 3-Hydroxypyridin, 6-Methyl-2-hydroxypyridin, 6-Methyl-3-hydroxypyridin, 4-Methylpyridin und 4-Chlorpyridin, ist,

X ein anionischer Monodentatligand ist, wie Halogen, Hydroxy, Pseudohalogen, $HSO_4$, $H_2PO_4$, Ascorbat oder $C_1$-$C_6$ Carboxylat, und

Y ein Anion, gewählt aus dem vorstehenden X oder Nitrat oder Perchlorat, ist.

2. Komplex nach Anspruch 1, worin $A_1$ und $A_2$ Ammoniak oder Alkylamin mit bis zu 6 Kohlenstoffatomen in der Alkylgruppe sind.

3. Komplex nach Anspruch 2, worin $A_1$ und $A_2$ Ammoniak sind.

4. Komplex nach einem der Ansprüche 1 bis 3, worin sowohl X als auch Y Halogenide sind.

5. Komplex nach Anspruch 4, worin das Halogenid Chlor ist.

6. Komplex nach einem der Ansprüche 1 bis 5, worin B 3-Hydroxypyridin ist.

7. Komplex nach einem der Ansprüche 1 bis 5, worin B 6-Methyl-2-hydroxypyridin ist.

8. Komplex nach einem der Ansprüche 1 bis 5, worin B 6-Methyl-3-hydroxypyridin ist.

9. Komplex nach einem der Ansprüche 1 bis 5, worin B 4-Methylpyridin ist.

10. Komplex nach einem der Ansprüche 1 bis 5, worin B 4-Chlorpyridin ist.

11. Pharmazeutische Zusammensetzung, umfassend einen aktiven Bestandteil, gemischt mit einem nichttoxischen, pharmazeutisch inerten Träger, wobei der aktive Bestandteil einen Triaminplatin(II)-Komplex mit der Formel:

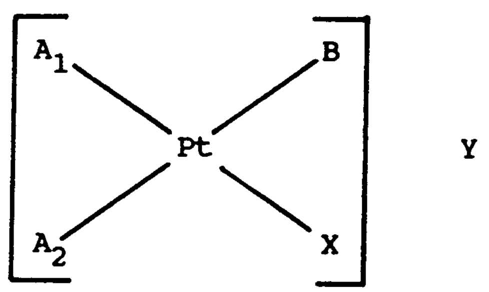

worin Pt in einem Wertigkeitszustand II ist und mit $A_1$ und $A_2$ in einer cis-Konfiguration koordiniert ist,

worin $A_1$ und $A_2$ gleich oder verschieden sein können und aus Ammoniak oder einem linearen, cyklischen oder verzweigten $C_1$-$C_6$ Alkylamin-Monodentat oder einem linearen, cyklischen oder verzweigten $C_1$-$C_6$ Alkylamin-Monodentat, das ein oder mehrere der folgenden Substituenten Halogen, Hydroxy, Alkoxy, Carboxy, Alkoxycarbonyl oder Alkylcarboxy trägt, gewählt werden,

B eine N-haltige heterocyklische oder substituierte heterocyklische Verbindung, gewählt aus der Gruppe, bestehend aus Pyridin, Pyrimidin, Pyridazin, Pyrazin, Imidazol, Pyrazol, Chinolin, Isochinolin oder Purin, oder eine der vorstehenden, die einen oder mehr Substituenten aus $C_1$-$C_6$ Alkyl, $C_1$-$C_6$

Alkoxy, Hydroxy, Carboxy, $C_1$-$C_6$ Alkoxycarbonyl, $C_1$-$C_6$ Alkylcarboxy, Halogen, Amin, Oxo, Nitrit, Nitro oder Amido, trägt, ist;

X ein anionischer Monodentatligand ist, wie Halogen, Hydroxy, Pseudohalogen, $HSO_4$, $H_2PO_4$, Ascorbat oder $C_1$-$C_6$ Carboxylat, und

Y ein Anion, gewählt aus dem vorstehenden X oder Nitrat oder Perchlorat, ist, umfaßt.

**12.** Zusammensetzung nach Anspruch 11, worin B Pyridin ist.

**13.** Zusammensetzung nach Anspruch 11, worin B ein substituiertes Pyridin ist.

**14.** Zusammensetzung nach Anspruch 13, worin B 3-Hydroxypyridin ist.

**15.** Zusammensetzung nach Anspruch 13, worin B 6-Methyl-2-hydroxypyridin ist.

**16.** Zusammensetzung nach Anspruch 13, worin B 6-Methyl-3-hydroxypyridin ist.

**17.** Zusammensetzung nach Anspruch 13, worin B 4-Methylpyridin ist.

**18.** Zusammensetzung nach Anspruch 13, worin B 4-Chlorpyridin ist.

**19.** Zusammensetzung nach Anspruch 13, worin B Cytosin ist.

**20.** Zusammensetzung nach Anspruch 13, worin B 2-Hydroxypyridin ist.

**21.** Zusammensetzung nach einem der Ansprüche 11 bis 20, worin sowohl X als auch Y Chlor sind.

**22.** Zusammensetzung nach einem der Ansprüche 11 bis 21 für die parenterale Verabreichung.

**23.** Zusammensetzung nach einem der Ansprüche 11 bis 21 für die orale Verabreichung.

**24.** Zusammensetzung nach einem der Ansprüche 11 bis 23 für die Verabreichung in einer Einfachdosis.

**25.** Zusammensetzung nach einem der Ansprüche 11 bis 23 für die Verabreichung in Mehrfachdosen.

**26.** Triaminplatinkomplex der allgemeinen Formel:

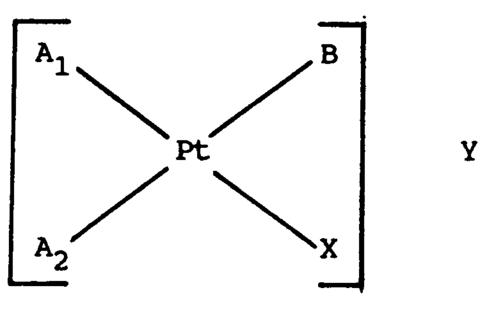

worin Pt in einem Wertigkeitszustand II ist und mit $A_1$ und $A_2$ in einer cis-Konfiguration koordiniert ist und

worin $A_1$, $A_2$, B, X und Y wie in einem der Ansprüche 11 bis 20 definiert sind, zur Verwendung in der Therapie.

**27.** Verwendung eines Triaminplatinkomplexes der allgemeinen Formel gemäß Anspruch 26 zur Herstellung eines Medikaments zur Behandlung von bösartigen Tumorzellen bei Tieren.

**28.** Verfahren zur Herstellung eines Platinkomplexes nach Anspruch 1, umfassend die Umsetzung einer

Verbindung der allgemeinen Formel

$PtA_1A_2XY$

mit einer Verbindung der allgemeinen Formel B,

worin Pt in einem Wertigkeitszustand II ist und mit $A_1$ und $A_2$ in einer cis-Konfiguration koordiniert ist und

worin $A_1$, $A_2$, X, Y und B wie in einem der Ansprüche 1 bis 10 definiert sind.

**29.** Verfahren nach Anspruch 28, worin die Verbindung der allgemeinen Formel

$PtA_1A_2XY$

durch die Umsetzung einer Verbindung der allgemeinen Formel

$PtA_1A_2X_2$

mit einem Silbersalz der allgemeinen Formel AgY hergestellt wird.

**30.** Verfahren zur Herstellung einer Zusammensetzung nach einem der Ansprüche 11 bis 25, bei dem der nichttoxische pharmazeutisch inerte Träger mit dem aktiven Bestandteil gemischt wird.

FIG. 1

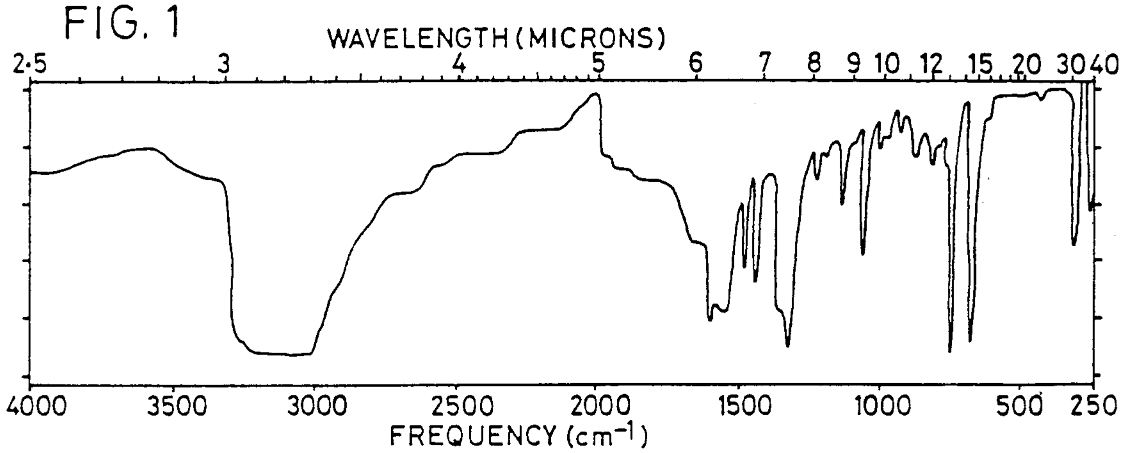


FIG. 2

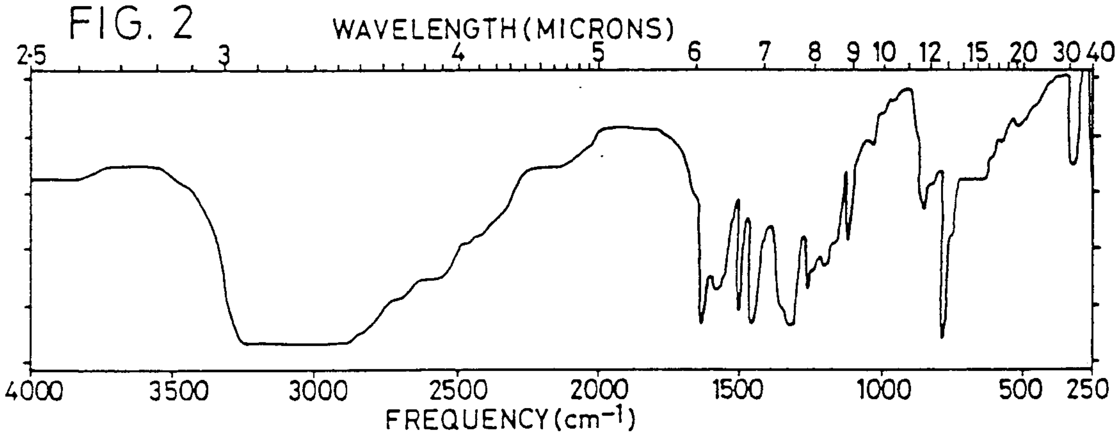


FIG. 3

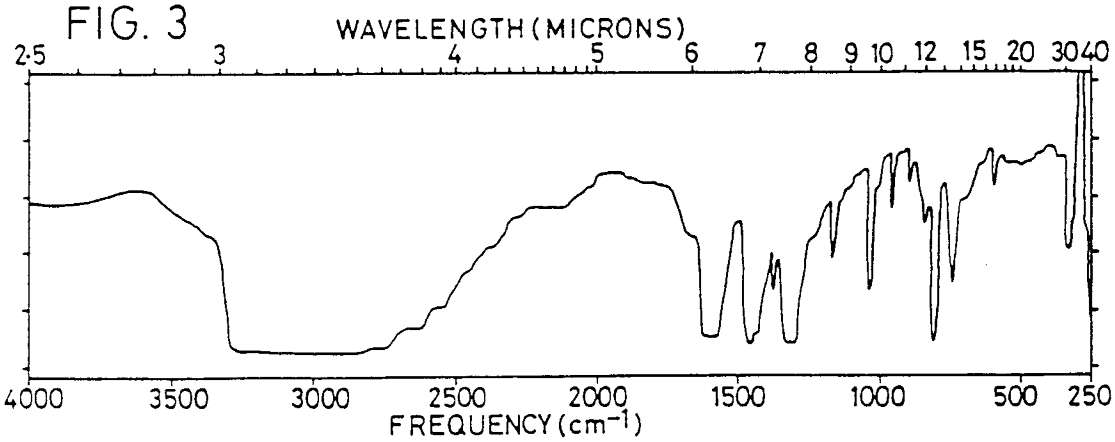


FIG. 4

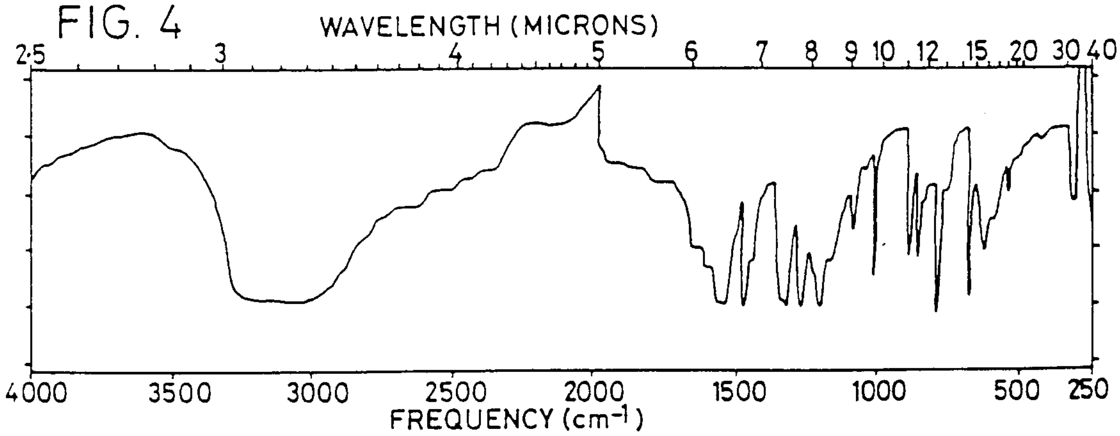

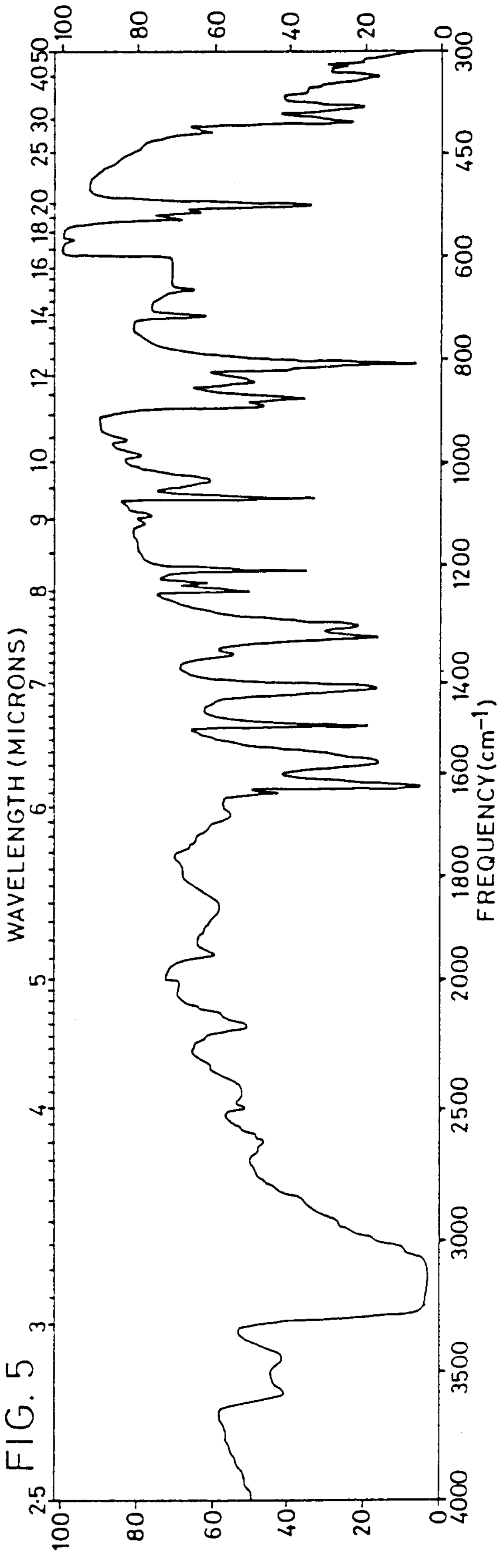
WAVELENGTH (MICRONS)
FREQUENCY (cm-1)
FIG. 5

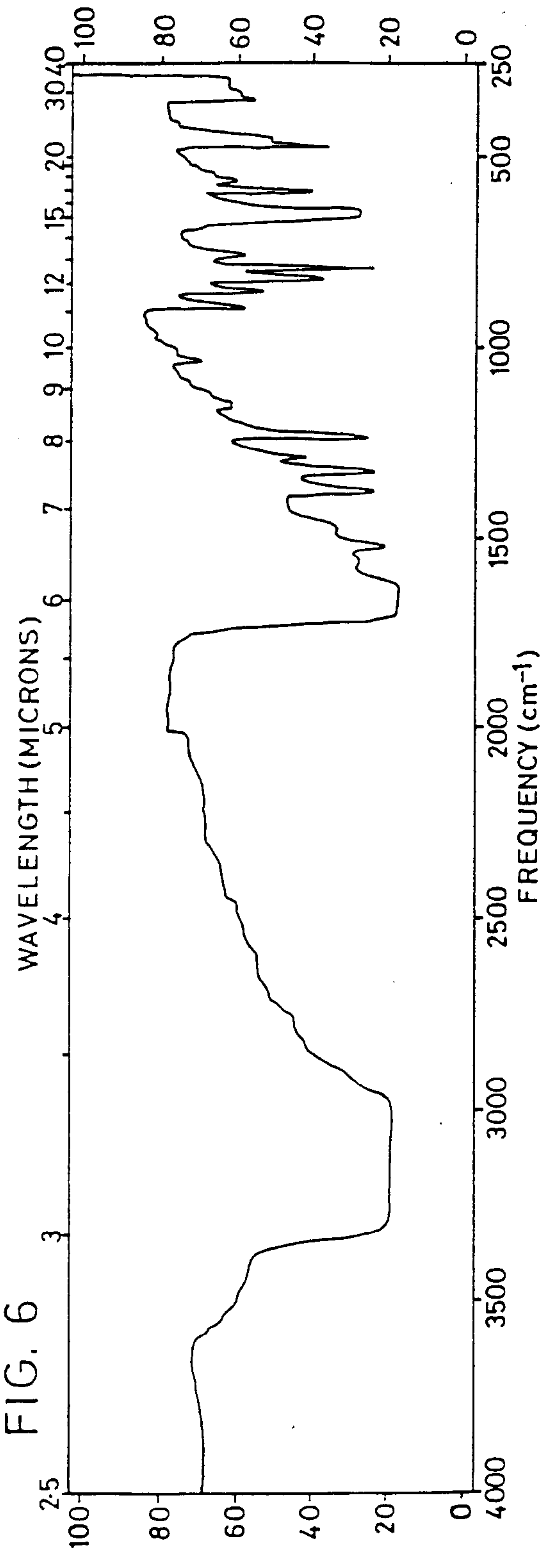
WAVELENGTH (MICRONS)
FREQUENCY (cm-1)
FIG. 6

FIG. 7

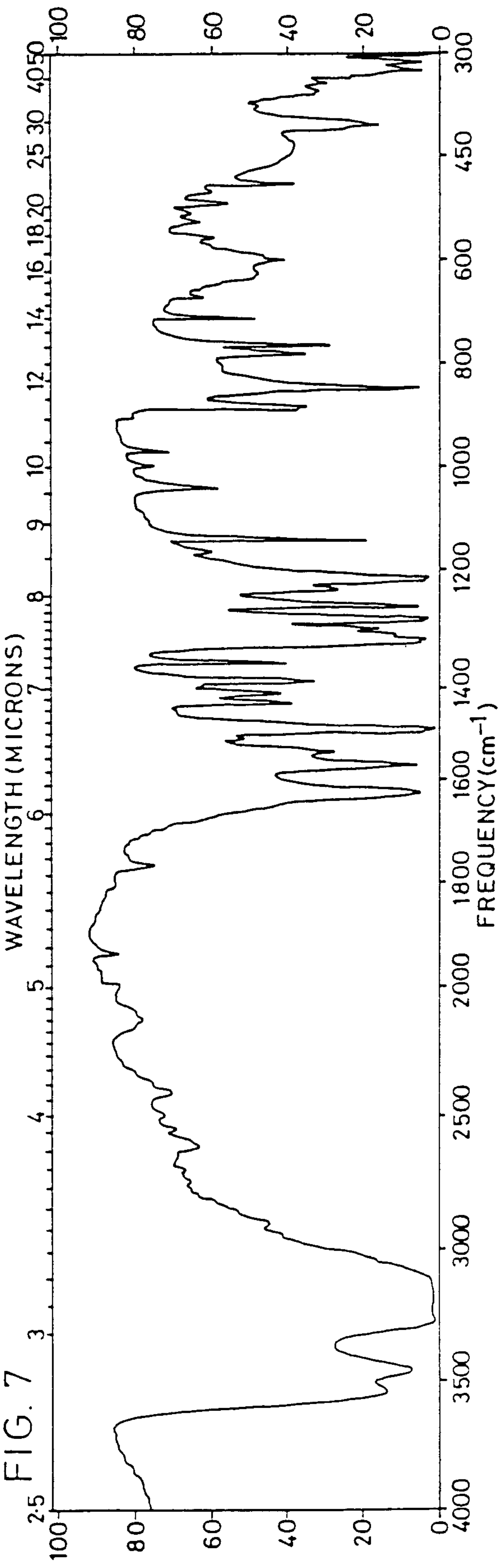
WAVELENGTH (MICRONS)
FREQUENCY ($cm^{-1}$)

FIG. 8

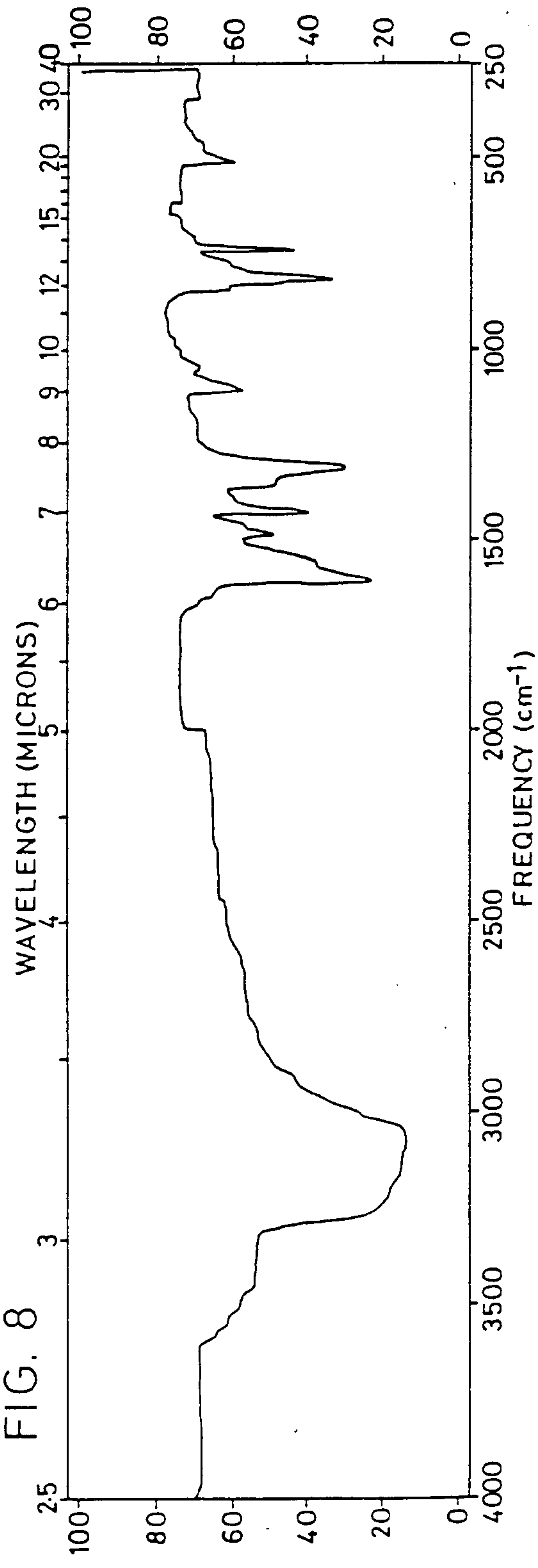
WAVELENGTH (MICRONS)
FREQUENCY ($cm^{-1}$)

FIG. 9

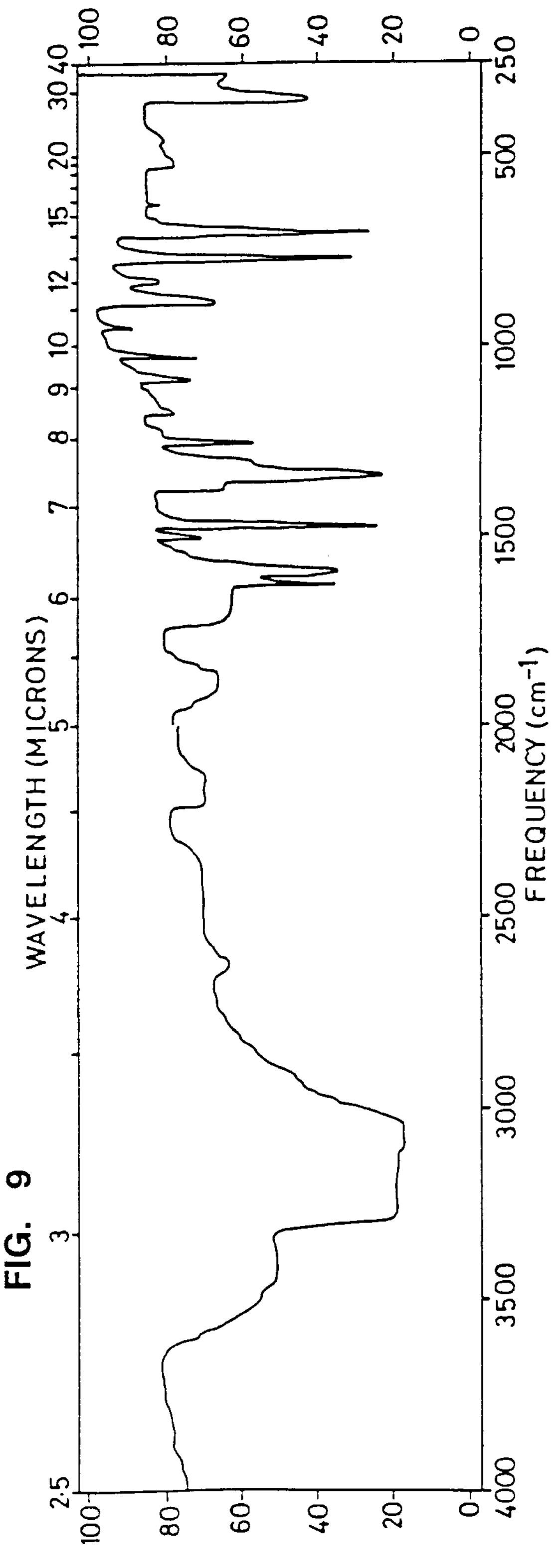
WAVELENGTH (MICRONS)
2·5
3
4
5
6
7
8
9
10
11
12
15
20
30
40
100
80
60
40
20
0
4000
3500
3000
2500
2000
1500
1000
500
250
FREQUENCY (cm⁻¹)